# EUROPÄISCHE PATENTSCHRIFT

(11) **EP 4 099 960 B1**
(45) Veröffentlichungstag und Bekanntmachung des Hinweises auf die Patenterteilung: **23.07.2025**
(21) Anmeldenummer: 21704465.0
(22) Anmeldetag: 05.02.2021
(51) Int. Cl.: A61F 2/44, A61F 2/30

(54) **ZWISCHENWIRBELIMPLANTAT**
INTERVERTEBRAL IMPLANT
IMPLANT INTERVERTÉBRAL

(30) Priorität: 06.02.2020 DE 102020103015
(43) Veröffentlichungstag der Anmeldung: 14.12.2022
(73) Patentinhaber: Aesculap AG, 78532 Tuttlingen (DE)
(72) Erfinder: KLINGSEIS, Susanne, 88400 Biberach (DE); MARX, Irene, 78647 Trossingen (DE); GRAF, Patricia, 78532 Tuttlingen (DE); RECK, Julian, 72516 Scheer (DE); KNUBBEN-BELIK, Marie, 78628 Rottweil (DE); KLEINWECHTER, Mandy, 78532 Tuttlingen (DE)
(74) Vertreter: Hoeger, Stellrecht & Partner Patentanwälte mbB
(86) Internationale Anmeldenummer: PCT/EP2021/052775
(87) Internationale Veröffentlichungsnummer: WO 2021/156423

(56) Entgegenhaltungen:
- US-A1- 2017 156 880
- US-A1- 2019 151 114
- US-A1- 2019 254 840

## Beschreibung

Die vorliegende Erfindung betrifft ein Zwischenwirbelimplantat zum Einsetzen in einen Bandscheibenraum zwischen zwei benachbarte Wirbelkörper einer menschlichen oder tierischen Wirbelsäule, wobei das Zwischenwirbelimplantat eine Implantatoberseite, welche eine erste Wirbelkörperanlagefläche zum Anlegen an einen ersten Wirbelkörper definiert, und eine Implantatunterseite, welche eine zweite Wirbelkörperanlagefläche zum Anlegen an einen zweiten Wirbelkörper definiert, aufweist, wobei das Zwischenwirbelimplantat eine Rahmenstruktur mit mindestens zwei Stützelementen umfasst, wobei sich die mindestens zwei Stützelemente von der Implantatoberseite bis zur Implantatunterseite erstrecken und wobei die mindestens zwei Stützelemente Stützelementlängsachsen definieren, die quer, insbesondere senkrecht, zur ersten und/oder zweiten Wirbelköperanlagefläche verlaufen. Die Erfindung ist im unabhängigen Anspruch 1 definiert. Vorteilhafte Ausgestaltungen sind in den abhängigen Ansprüchen dargestellt.

Zwischenwirbelimplantate der eingangs beschriebenen Art sind in verschiedenen Ausführungsformen bekannt, beispielsweise aus der US 2019/0254840 A1. Sie werden als Ersatz für eine geschädigte Bandscheibe zwischen zwei benachbarte Wirbelkörper in das zugeordnete Bandscheibenfach eingesetzt, um die benachbarten Wirbelkörper miteinander zu verbinden und so die Wirbelsäule auf einem zwei Wirbelkörper umfassenden Abschnitt zu versteifen. Auf das Zwischenwirbelimplantat wirken große Kräfte, die von den benachbarten Wirbelkörpern auf die Implantatoberseite und die Implantatunterseite ausgeübt werden. Es ist daher wichtig, dass zum Erhalt der Höhe des Bandscheibenfachs, also des Abstands zwischen den benachbarten Wirbelkörpern, das Zwischenwirbelimplantat eine ausreichende Stabilität aufweist, um eine Verletzung der im Spinalkanal verlaufenden Nerven zu vermeiden. Insbesondere bei offenporigen Strukturen, die als Zwischenwirbelimplantate genutzt werden, besteht hier relativ leicht die Gefahr, dass sich das Zwischenwirbelimplantat durch Kompression insgesamt verformt und so eine Höhe des Bandscheibenfachs nicht erhalten werden kann. Unerwünschte Folge hiervon kann insbesondere eine Schädigung von zwischen den Wirbelkörpern austretenden Gefäßen und Nerven sowie im Spinalkanal verlaufender Nerven sein.

Aus der US 2019/0151114 A1 ist ein Implantat mit verbessertem Knochenkontakt bekannt. Ein poröses, zwischen Wirbel einsetzbares Abstandselement ist in der US 2017/0156880 A1 beschrieben.

Es ist daher eine Aufgabe der vorliegenden Erfindung, ein Zwischenwirbelimplantat der eingangs beschriebenen Art so zu verbessern, dass es eine ausreichende Stabilität aufweist.

Diese Aufgabe wird bei einem Zwischenwirbelimplantat der eingangs beschriebenen Art erfindungsgemäß dadurch gelöst, dass die Rahmenstruktur einen ersten in sich geschlossenen inneren Rahmenteil und einen zweiten in sich geschlossenen inneren Rahmenteil umfasst, dass die mindestens zwei Stützelemente die beiden inneren Rahmenteile miteinander verbinden durch eine direkte Anbindung der inneren Rahmenteile an die mindestens zwei Stützelemente.

Eine solche Rahmenstruktur mit den mindestens zwei Stützelementen vorzusehen hat insbesondere den Vorteil, dass auf die Implantatoberseite und die Implantatunterseite einwirkende Kräfte direkt in die mindestens zwei Stützelemente eingeleitet werden können. Eine Kompression des Zwischenwirbelimplantats und somit eine Abnahme der Höhe des Bandscheibenfachs, in das das Zwischenwirbelimplantat eingesetzt ist, kann so deutlich besser vermieden werden. Zudem ermöglichen es die mindestens zwei Stützelemente insbesondere auch, an ihren Enden, die beispielsweise bis zur Implantatoberseite und bis zur Implantatunterseite reichen können, Verankerungsvorsprünge, sogenannte Spikes, anzuordnen. Diese können somit optimal insbesondere an Zwischenwirbelimplantate angebunden werden, die überwiegend aus einer offenporigen Gitterstruktur bestehen, welche beispielsweise durch additive oder generative Herstellungsverfahren ausgebildet werden kann. Eine offenporige Struktur ist insbesondere wünschenswert, um das Einwachsen von Knochen in das Zwischenwirbelimplantat zu ermöglichen. Überdies erleichtern die Stützelemente das Reinigen des Zwischenimplantats insbesondere nach dessen Herstellung, und zwar beispielsweise dann, wenn das Zwischenwirbelimplantat eine offenporige Gitterstruktur umfasst. Spülschatten, die üblicherweise entstehen, wenn die Verankerungsvorsprünge direkt an der offenporigen Gitterstruktur angebracht werden, werden durch die Anordnung auf den Stützelementen für das Auswaschen des Zwischenwirbelimplantats nach der Fertigung, beispielsweise zur Entfernung eventueller Hilfsmittelrückstände, weitestgehend verringert. Die Rahmenstruktur kann insbesondere mehrteilig ausgebildet sein, wobei die mindestens zwei Stützelemente die mehreren Komponenten der Rahmenstruktur miteinander verbinden können, um so eine solide und stabile Struktur des Zwischenwirbelimplantats insgesamt zu erhalten. Die mindestens zwei Stützelemente können insbesondere stabförmig ausgebildet sein. Ferner können die mindestens zwei Stützelemente eine Querschnittsfläche definieren, die längs ihrer Streckung konstant oder im Wesentlichen konstant ist. Dies dient insbesondere der Verringerung von Spülschatten bei der Reinigung des Zwischenwirbelimplantats. Durch die Durchgängigkeit der mindestens zwei Stützelemente wird eine Reinigung des Zwischenwirbelimplantats, insbesondere einer von diesem optional umfassten offenporigen Gitterstruktur, erleichtert. Für das Auswaschen des Zwischenwirbelimplantats nach der Fertigung, beispielsweise zur Entfernung etwaiger Hilfsmittelrückstände, können Spülschatten soweit als möglich reduziert werden. Gemäß der Erfindung ist vorgesehen, dass die Rahmenstruktur einen ersten in sich geschlossenen inneren Rahmenteil und einen zweiten in sich geschlossenen inneren Rahmenteil umfasst und dass die mindestens zwei Stützelemente die beiden inneren Rahmenteile miteinander verbinden. Durch eine solche direkte Anbindung der inneren Rahmenteile an die mindestens zwei Stützelemente kann eine Bewegung der inneren Rahmenteile aufeinander zu vermieden werden. Insgesamt kann so also eine Stabilität des Zwischenwirbelimplantats erhöht werden. Insbesondere können auch mehr als zwei innere Rahmenteile vorgesehen sein, um eine Verwindungssteifigkeit des Zwischenwirbelimplantats weiter zu verbessern.

Vorteilhaft ist es, wenn die mindestens zwei Stützelemente vom Zwischenwirbelimplantat weg weisende Endflächen aufweisen und wenn mindestens eine dieser Endflächen mindestens einen vom Zwischenwirbelimplantat weg weisenden Verankerungsvorsprung trägt. Ein solcher Verankerungsvorsprung, auch als Spike bezeichnet, dient zur sicheren Positionierung des Zwischenwirbelimplantats im Bandscheibenfach. Die Verankerungsvorsprünge können sich in die benachbarten Wirbelkörper eingraben und so eine Relativbewegung zwischen dem Zwischenwirbelimplantat und den benachbarten Wirbelkörpern verhindern oder zumindest begrenzen. Durch das Anordnen der Verankerungsvorsprünge an den Endflächen der Stützelemente ist eine zuverlässige und solide Anbindung derselben an das Zwischenwirbelimplantat möglich. Ein Abscheren der Verankerungsvorsprünge vom Zwischenimplantat kann dadurch sicher verhindert werden. Diese Gefahr besteht insbesondere dann, wenn solche Verankerungsvorsprünge direkt auf offenporigen Gitterstrukturen angeordnet werden. Hier kann es zu einer Verformung der Gitterstruktur und somit zu einer Funktionsunfähigkeit der Verankerungsvorsprünge kommen, sei es dass die Verankerungsvorsprünge in das Zwischenwirbelimplantat hineingedrückt oder umgebogen werden, so dass sie nicht mehr in Richtung auf die benachbarten Wirbelkörper weisen und in diese zum Verankern des Zwischenwirbelimplantats eindringen können.

Um eine sichere Verankerung des Zwischenwirbelimplantats an den benachbarten Wirbelkörpern zu ermöglichen, ist es günstig, wenn der mindestens eine Verankerungsvorsprung spitz, insbesondere dornartig, ausgebildet ist.

Auf einfache Weise lässt sich das Zwischenwirbelimplantat ausbilden, wenn der mindestens eine Verankerungsvorsprung kegelförmig oder im Wesentlichen kegelförmig ausgebildet ist. Er weist dann eine zum Eingraben in die benachbarten Wirbelkörper hilfreiche Spitze auf und kann zudem mit seiner Grundfläche großflächig an das zugeordnete Stützelement angebunden werden.

Günstig ist es, wenn der mindestens eine Verankerungsvorsprung eine Vorsprunglängsachse definiert und wenn die Vorsprunglängsachse parallel oder im Wesentlichen parallel zur Stützelementlängsachse des zugeordneten Stützelements verläuft. Durch die parallele oder kollineare Ausrichtung der Vorsprunglängsachse und der Stützelementlängsachse des jeweiligen Stützelements kann eine Stabilität des Zwischenwirbelimplantats und insbesondere eine Anbindung des mindestens einen Verankerungsvorsprungs an das zugeordnete Stützelement verbessert werden.

Vorteilhaft ist es, wenn die mindestens zwei Stützelemente einen Querschnitt bezogen auf die jeweilige Stützelementlängsachse definieren, welcher rechteckig, dreieckig oder kreisförmig oder durch eine Kombination aus einem Rechteck und einem Halbkreis gebildet ist. Stützelemente mit derartigen Querschnittsformen lassen sich auf einfache Weise ausbilden. Sie weisen für den ihnen zugedachten Zweck eine hinreichende Stabilität auf. Insbesondere können sie aufgrund ihrer Ausgestaltung mit einer Rahmenstruktur einfach und sicher verbunden werden.

Um die Stabilität des Zwischenwirbelimplantats zu verbessern, ist es günstig, wenn das Zwischenwirbelimplantat drei oder vier Stützelemente umfasst. Diese können insbesondere symmetrisch bezogen auf eine Symmetrieebene des beispielsweise spiegelsymmetrisch ausgebildeten Zwischenwirbelimplantats angeordnet sein.

Zur Erhöhung der Stabilität des Zwischenwirbelimplantats ist es günstig, wenn die mindestens zwei Stützelemente massiv ausgebildet sind. Massiv bedeutet insbesondere, dass sie ohne Hohlräume ausgebildet sind und so massive Träger bilden, die eine Kompression zwischen der Implantatoberseite und der Implantatunterseite vermeiden helfen.

Gemäß einer weiteren bevorzugten Ausführungsform der Erfindung kann vorgesehen sein, dass das Zwischenwirbelimplantat eine zentrale Durchbrechung aufweist und dass sich die Durchbrechung von der Implantatoberseite bis zur Implantatunterseite durch das Zwischenwirbelimplantat hindurch erstreckt. Die Durchbrechung kann insbesondere genutzt werden, um Knochenwachstum förderndes Material aufzunehmen, wodurch das Einwachsen von Knochen in das Zwischenwirbelimplantat und somit eine Stabilität der Verbindung zwischen den benachbarten Wirbelkörpern, die das Zwischenwirbelimplantat miteinander verbindet, verbessert wird.

Auf einfache Weise lässt sich das Zwischenwirbelimplantat ausbilden, wenn die Durchbrechung hohlzylindrisch oder im Wesentlichen hohlzylindrisch ausgebildet ist oder einen ovalen oder rechteckigen, insbesondere quadratischen, Querschnitt aufweist. Je nach Größe und Außenkontur des Zwischenwirbelimplantats kann die Form der Durchbrechung und insbesondere deren Querschnittsform gewählt werden, um bei größtmöglicher Stabilität des Zwischenwirbelimplantats insgesamt ein optimales Einwachsen von Knochen in das Zwischenwirbelimplantat hinein zu ermöglichen.

Günstig ist es, wenn die Durchbrechung eine Durchbrechungslängsachse definiert und wenn die Durchbrechungslängsachse quer, insbesondere senkrecht, zur ersten Wirbelkörperanlagefläche und/oder quer, insbesondere senkrecht, zur zweiten Wirbelkörperanlagefläche verläuft. Insbesondere kann die Durchbrechungslängsachse parallel zu den Stützelementlängsachsen verlaufen. So kann insbesondere eine Stabilität des Zwischenwirbelimplantats weiter erhöht werden, unabhängig davon, welche Querschnittsform und Größe die Durchbrechung aufweist.

Besonders kompakt ausbilden lässt sich das Zwischenwirbelimplantat, wenn die beiden inneren Rahmenteile die Durchbrechung mindestens abschnittsweise begrenzen.

Auf einfache Weise lässt sich das Zwischenwirbelimplantat ausbilden, wenn der erste innere Rahmenteil in Form eines Rings ausgebildet ist und/oder wenn der zweite innere Rahmenteil in Form eines Rings ausgebildet ist. Die inneren Rahmenteile, die mit den mindestens zwei Stützelementen verbunden sind, definieren somit auch eine Position der Stützelemente am Zwischenwirbelimplantat.

Vorteilhaft ist es, wenn der erste innere Rahmenteil die Implantatoberseite mindestens abschnittsweise begrenzt und/oder wenn der zweite innere Rahmenteil die Implantatunterseite mindestens abschnittsweise begrenzt. Die inneren Rahmenteile können so eine äußere Kontur des Zwischenwirbelimplantats mindestens abschnittsweise begrenzen. Durch die Anbindung der mindestens zwei Stützelemente an die inneren Rahmenteile können so auf die inneren Rahmenteile wirkende Kräfte in die Stützelemente eingeleitet werden, was eine Stabilität des Zwischenwirbelimplantats weiter verbessern hilft.

Günstig ist es, wenn die Rahmenstruktur einen ersten in sich geschlossenen äußeren Rahmenteil und einen zweiten in sich geschlossenen äußeren Rahmenteil umfasst und wenn die beiden äußeren Rahmenteile mit den beiden inneren Rahmenteilen, insbesondere verwindungssteif, verbunden sind. Eine solche Rahmenstruktur kann insbesondere ein Implantatvolumen definieren, welches sich zwischen den inneren und äußeren Rahmenteilen erstreckt. Dieses Implantatvolumen kann beispielsweise durch eine offenporige Gitterstruktur ausgefüllt werden, die das Einwachsen von Knochen ermöglicht, jedoch nicht eine für die Funktion des Zwischenwirbelimplantats erforderliche Stabilität, insbesondere Kompressionsstabilität, aufweist. Die Rahmenstruktur dient bei dem Zwischenwirbelimplantat insbesondere dazu, die Stabilität des Zwischenwirbelimplantats sicherzustellen. So lässt sich die Funktion des optimalen Einwachsens von Knochengewebe beispielsweise in definierter Weise von einer Funktion zur Maximierung einer Stabilität des Zwischenwirbelimplantats gezielt trennen.

Günstig ist es, wenn der erste äußere Rahmenteil die Implantatoberseite mindestens abschnittsweise begrenzt und/oder wenn der zweite äußere Rahmenteil die Implantatunterseite mindestens abschnittsweise begrenzt. Die äußeren Rahmenteile können so auf das Zwischenwirbelimplantat einwirkende Kräfte auf die inneren Rahmenteile und damit indirekt auch auf die Stützelemente ableiten. Zudem können die äußeren Rahmenteile das Zwischenwirbelimplantat auch seitlich begrenzen. Beispielsweise können auch drei oder mehr äußere Rahmenteile vorgesehen sein, je nach Größe des Zwischenwirbelimplantats, das je nach Position des Bandscheibenfachs, in das es eingesetzt werden soll, eine entsprechende Größe aufweist.

Auf einfache Weise lässt sich das Zwischenwirbelimplantat ausbilden, wenn der erste äußere Rahmenteil in Form eines Rings ausgebildet ist und/oder wenn der zweite äußere Rahmenteil in Form eines Rings ausgebildet ist. Ein Ring muss nicht zwingend eine Kreisform aufweisen. Er kann insbesondere oval ausgebildet sein oder auch aus mehreren Abschnitten bestehen, die unterschiedliche Krümmungen aufweisen, insbesondere auch geradlinige Abschnitte umfassen.

Vorzugsweise umfasst das Zwischenwirbelimplantat einen Rahmengrundkörper, welcher die äußeren Rahmenteile mit den inneren Rahmenteilen verbindet. Der Rahmengrundkörper kann beispielsweise zur Handhabung des Zwischenwirbelimplantats genutzt werden. Zudem kann er durch entsprechende Ausgestaltung einen wesentlichen Beitrag zur Stabilität des Zwischenwirbelimplantats leisten.

Um insbesondere eine hohe Stabilität des Zwischenwirbelimplantats erreichen zu können, ist es vorteilhaft, wenn der Rahmengrundkörper massiv ausgebildet ist.

Auf einfache Weise lässt sich das Zwischenwirbelimplantat ausbilden, wenn der Rahmengrundkörper quaderförmig oder im Wesentlichen quaderförmig ausgebildet ist.

Zur Handhabung des Zwischenwirbelimplantats ist es insbesondere günstig, wenn es eine Instrumentenaufnahme zum kraft- und/oder formschlüssigen in Eingriff Bringen mit einem Einsetzinstrument umfasst.

Eine Handhabung des Zwischenwirbelimplantats lässt sich einfach und sicher realisieren, wenn die Instrumentenaufnahme am Rahmengrundkörper angeordnet oder ausgebildet ist. Insbesondere kann die Instrumentenaufnahme von einer Vorderseite her, die sich zwischen der Implantatoberseite und der Implantatunterseite quer zu diesen erstreckt, zugänglich sein.

Vorteilhaft ist es, wenn die Instrumentenaufnahme eine Instrumentenaufnahmelängsachse definiert und wenn die Instrumentenaufnahmelängsachse quer, insbesondere senkrecht, zur Durchbrechungslängsachse verläuft. Dies ermöglicht es insbesondere, das Zwischenwirbelimplantat mit einem Einsetzinstrument in Eingriff zu Bringen und seitlich in ein Bandscheibenfach einzuschieben. Sowohl die Implantatunterseite als auch die Implantatoberseite können dabei völlig frei bleiben, so dass das Zwischenwirbelimplantat ungehindert in das Bandscheibenfach eingeführt werden kann.

Auf einfache Weise lässt sich das Zwischenwirbelimplantat ausbilden, wenn die Instrumentenaufnahme in Form einer Bohrung oder durch eine Hülse ausgebildet ist.

Eine Kopplung des Zwischenwirbelimplantats zum Einsetzen desselben in ein Bandscheibenfach mit einem Einsetzinstrument lässt sich auf einfache Weise dadurch verbessern, wenn die Instrumentenaufnahme mit einem Innengewinde versehen ist. Das Einsetzinstrument kann dann mit einem Außengewinde versehen sein, welches zum Innengewinde der Instrumentenaufnahme korrespondiert. So wird ein Verschrauben des Einsetzinstruments mit dem Zwischenwirbelimplantat ermöglicht, wodurch eine stabile Verbindung zwischen dem Einsetzinstrument und dem Zwischenwirbelimplantat temporär herstellbar ist.

Gemäß einer weiteren bevorzugten Ausführungsform der Erfindung kann vorgesehen sein, dass sich eine Rückseite des Zwischenwirbelimplantats quer, insbesondere senkrecht, zur ersten Wirbelkörperanlagefläche und quer, insbesondere senkrecht, zur zweiten Wirbelkörperanlagefläche erstreckt. So lässt sich insgesamt beispielsweise ein quaderförmiges oder im Wesentlichen quaderförmiges Zwischenwirbelimplantat ausbilden.

Vorzugsweise erstreckt sich die Instrumentenaufnahme von der Vorderseite des Zwischenwirbelimplantats bis zur Durchbrechung, insbesondere parallel oder im Wesentlichen parallel zur Implantatoberseite und/oder zur Implantatunterseite. Dies ermöglicht insbesondere die Ausbildung oder Anordnung der Instrumentenaufnahme ohne Stabilitätseinbußen am Zwischenwirbelimplantat. Der Rahmengrundkörper kann insbesondere bis auf die Instrumentenaufnahme massiv ausgebildet sein, um eine möglichst hohe Stabilität des Zwischenwirbelimplantats sicherstellen zu können.

Vorzugsweise sind die erste Wirbelkörperanlagefläche und/oder die zweite Wirbelkörperanlagefläche mindestens abschnittsweise, insbesondere vollständig, eben oder im Wesentlichen eben ausgebildet sind. Sie können so optimal an eine das Bandscheibenfach begrenzende Seitenfläche des Wirbelkörpers angepasst werden, um eine optimale Lastaufnahme zu ermöglichen.

Günstig ist es, wenn die erste Wirbelkörperanlagefläche eine erste Anlageebene definiert und wenn die zweite Wirbelkörperanlagefläche eine zweite Anlageebene definiert und wenn die erste Anlageebene und die zweite Anlageebene parallel zueinander verlaufen oder um einen Neigungswinkel gegeneinander geneigt sind. Diese Ausgestaltung ermöglicht es insbesondere, das Zwischenwirbelimplantat optimal an das physiologisch vorgegebene Bandscheibenfach anzupassen, um die benachbarten Wirbelkörper in einem gewünschten Abstand und in einer natürlichen Orientierung relativ zueinander zu halten.

Vorzugsweise weist der Neigungswinkel einen Wert in einem Bereich zwischen 0° und etwa 20° auf. Ein solcher Neigungswinkel ermöglicht es insbesondere, das Zwischenwirbelimplantat optimal an die physiologischen Gegebenheiten der Wirbelsäule des Patienten anzupassen.

Gemäß einer weiteren bevorzugten Ausführungsform der Erfindung kann vorgesehen sein, dass die erste Wirbelkörperanlagefläche und/oder die zweite Wirbelkörperanlagefläche mindestens abschnittsweise, insbesondere vollständig, vom Zwischenwirbelimplantat weg weisend konvex gekrümmt, insbesondere zwei- oder dreidimensional gekrümmt, ausgebildet sind. Die Wirbelkörperanlagenflächen in der beschriebenen Weise auszubilden ermöglicht insbesondere eine optimale Anpassung an natürliche Gegebenheiten einer Bandscheibe eines Patienten, dem das Zwischenwirbelimplantat in ein ausgeräumtes Bandscheibenfach eingesetzt werden soll.

Um das Einwachsen von Knochengewebe in das Zwischenwirbelimplantat hinein zu erleichtern und zu verbessern, ist es günstig, wenn ein vom Zwischenwirbelimplantat definiertes Implantatvolumen mindestens teilweise durch eine offenporige Gitterstruktur ausgefüllt ist. Insbesondere kann das Implantatvolumen durch eine solche offenporige Gitterstruktur vollständig ausgefüllt sein. Die Gitterstruktur kann insbesondere durch ein drahtartiges Gewebe oder eine Gewebestruktur ausgebildet sein, die eine Vielzahl von Hohlräumen, die miteinander in Fluidverbindung stehen, aufweist. Eine solche Gitterstruktur kann beispielweise auf einfache Weise durch ein generatives Herstellungsverfahren ausgebildet werden.

Günstigerweise ist die Gitterstruktur durch ein generatives Herstellungsverfahren ausgebildet. Dies ermöglicht es insbesondere, das Zwischenwirbelimplantat aus einem Stück, also insbesondere monolithisch, auszubilden, und zwar sowohl die tragenden Teile des Zwischenwirbelimplantats, also insbesondere die Rahmenstruktur, als auch diejenigen Teile, die ein Einwachsen von Knochengewebe in das Zwischenwirbelimplantat hinein erleichtern, wie insbesondere eine offenporige Gitterstruktur. Die Angabe "generativ" wird in der vorliegenden Anmeldung synonym zur Angabe "additiv" verwendet.

Die Herstellung des Zwischenwirbelimplantats lässt sich insgesamt verbessern, wenn das Zwischenwirbelimplantat insgesamt und/oder die Rahmenstruktur durch ein generatives Herstellungsverfahren ausgebildet sind. Insbesondere kann auch eine offenporige Gitterstruktur des Zwischenwirbelimplantats, wenn eine solche vorgesehen ist, durch das generative Herstellungsverfahren ausgebildet werden.

Günstigerweise sind die Rahmenstruktur und/oder die Gitterstruktur und und/oder das Zwischenwirbelimplantat insgesamt durch selektives Lasersintern ausgebildet. Das Zwischenwirbelimplantat lässt sich so beispielsweise schichtweise aufbauen. Es können insbesondere beliebige Strukturen ausgebildet werden.

Gemäß einer weiteren bevorzugten Ausführungsform der Erfindung kann vorgesehen sein, dass die Rahmenstruktur und/oder das Zwischenwirbelimplantat insgesamt aus einem metallischen Werkstoff und/oder aus einem Kunststoff ausgebildet sind. Insbesondere kann auch die Gitterstruktur, welche offenporig ausgebildet sein kann, aus einem metallischen Werkstoff und/oder aus einem Kunststoff ausgebildet sein.

Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische Gesamtansicht eines ersten Ausführungsbeispiels eines Zwischenwirbelimplantats;
- Figur 2:: eine Draufsicht auf das Zwischenwirbelimplantat aus Figur 1;
- Figur 3:: eine Schnittansicht längs Linie 3-3 in Figur 2;
- Figur 4:: eine Schnittansicht längs Linie 4-4 in Figur 2;
- Figur 5:: eine schematische perspektivische Gesamtansicht eines weiteren Ausführungsbeispiels eines Zwischenwirbelimplantats;
- Figur 6:: eine Draufsicht auf das Zwischenwirbelimplantat aus Figur 5;
- Figur 7:: eine Schnittansicht längs Linie 7-7 in Figur 6;
- Figur 8:: eine Schnittansicht längs Linie 8-8 in Figur 6;
- Figur 9:: eine schematische Seitenansicht zweier benachbarter Wirbel einer menschlichen Wirbelsäule mit einem in ein Bandscheibenfach zwischen den Wirbeln eingesetztes Zwischenwirbelimplantat;
- Figur 10:: eine schematische perspektivische Gesamtansicht des Zwischenwirbelimplantats aus Figur 9;
- Figur 11:: eine Ansicht des Zwischenwirbelimplantats aus Figur 10 von hinten in Richtung des Pfeils A aus Figur 12;
- Figur 12:: eine Draufsicht auf das Zwischenwirbelimplantat aus Figur 10 von oben;
- Figur 13:: eine Ansicht des Zwischenwirbelimplantats aus Figur 10 von vorn in Richtung des Pfeils B aus Figur 12;
- Figur 14:: eine Schnittansicht des Zwischenwirbelimplantats aus Figur 12 längs Linie 14-14; und
- Figur 15:: eine Schnittansicht des Zwischenwirbelimplantats aus Figur 11 längs Linie 15-15.

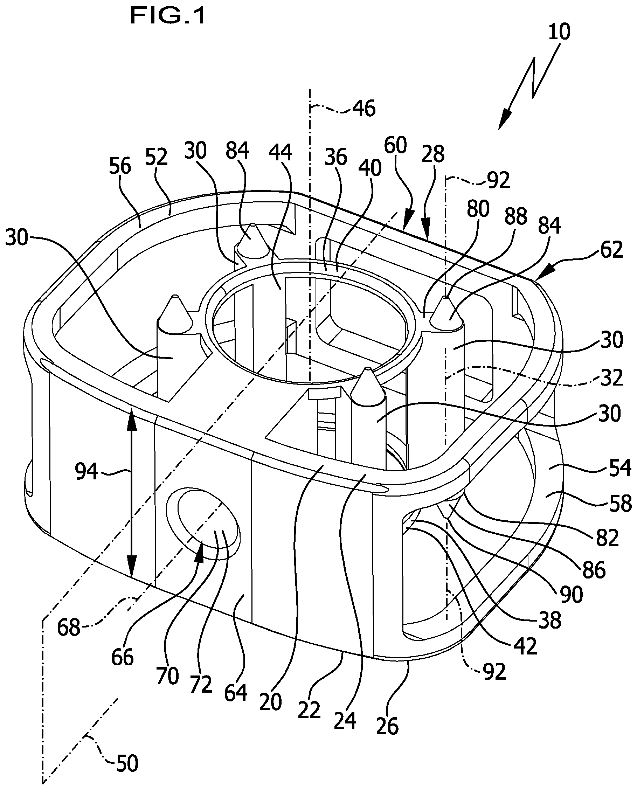

In Figur 1 ist eine schematische perspektivische Gesamtansicht eines ersten Ausführungsbeispiels eines insgesamt mit dem Bezugszeichen 10 bezeichneten Zwischenwirbelimplantats dargestellt.

Das Zwischenwirbelimplantat 10 ist ausgebildet zum Einsetzen in einen Bandscheibenraum 12 zwischen zwei benachbarte Wirbelkörper 14 und 16 einer menschlichen oder tierischen Wirbelsäule 18.

Das Zwischenwirbelimplantat 10 weist eine Implantatoberseite 20 und eine Implantatunterseite 22 auf.

Die Implantatoberseite 20 definiert eine erste Wirbelkörperanlagefläche 24 zum Anlegen an den Wirbelkörper 14. Die Implantatunterseite 22 definiert eine zweite Wirbelkörperanlagefläche 26 zum Anlegen an den Wirbelkörper 16.

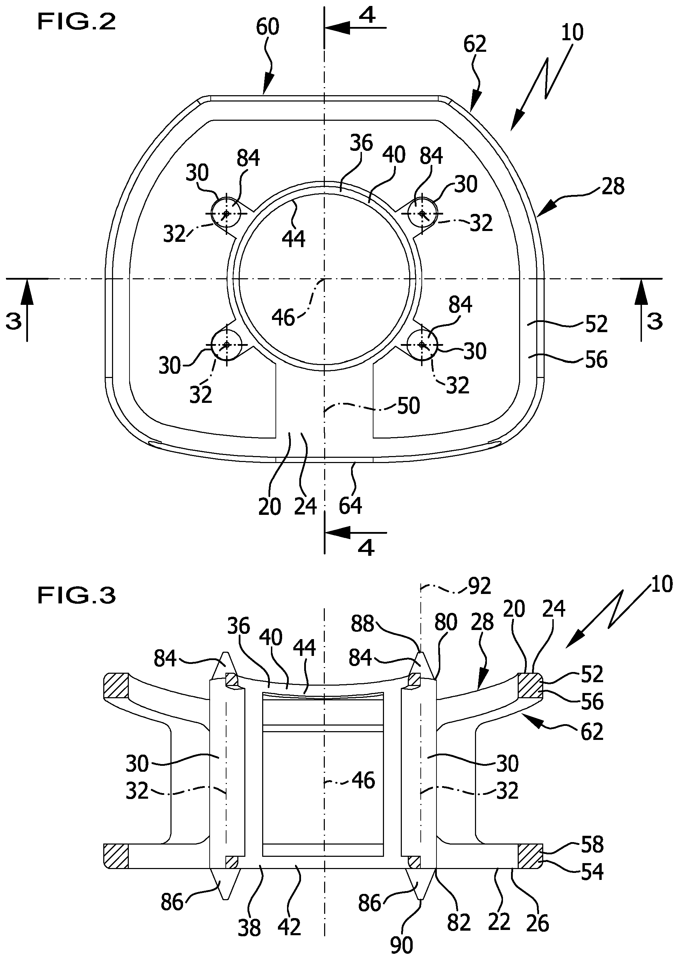

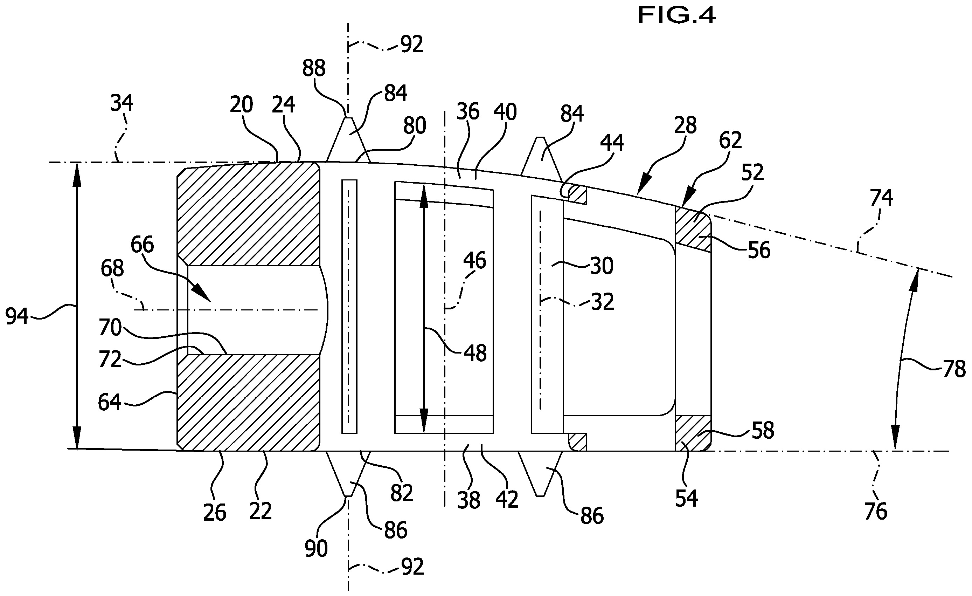

Das Zwischenwirbelimplantat 10 umfasst ferner eine Rahmenstruktur 28 mit mindestens zwei Stützelementen 30. Bei dem in den Figuren 1 bis 4 dargestellten Ausführungsbeispiel des Zwischenwirbelimplantats 10 sind insgesamt vier Stützelemente 30 vorgesehen.

Die Stützelemente 30 erstrecken sich von der Implantatoberseite 20 bis zur Implantatunterseite 22.

Die Stützelemente 30 definieren Stützelementlängsachsen 32, die quer zu den Wirbelkörperanlageflächen 24 und 26 verlaufen. Bei dem in den Figuren 1 bis 4 dargestellten Ausführungsbeispiel erstrecken sich die Stützelementlängsachsen 32 senkrecht zur zweiten Wirbelkörperanlagefläche 26. Ferner verlaufen die Stützelementlängsachsen 32 senkrecht zu einer an die vom Zwischenwirbelimplantat 10 weg weisend konvex gekrümmte erste Wirbelkörperanlagefläche 24 anliegende Tangentialebene 34.

Die Rahmenstruktur 28 umfasst einen ersten in sich geschlossenen inneren Rahmenteil 36 und einen zweiten in sich geschlossenen inneren Rahmenteil 38. Die Stützelemente 30 verbinden die beiden Rahmenteile 36 und 38 miteinander. Die Rahmenteile 36 und 38 sind jeweils in Form eines Rings 40 beziehungsweise 42 ausgebildet.

Der erste innere Rahmenteil 36 bildet einen Teil der Implantatoberseite 20 beziehungsweise begrenzt diese abschnittsweise, und zwar in Form des Rings 40. In analoger Weise begrenzt der zweite innere Rahmenteil 38 die Implantatunterseite 22 abschnittsweise beziehungsweise bildet einen Teil der Implantatunterseite 22.

Das Zwischenwirbelimplantat 10 weist eine zentrale Durchbrechung 44 auf, welche sich von der Implantatoberseite 20 bis zur Implantatunterseite 22 durch das Zwischenwirbelimplantat 10 hindurch erstreckt. Die Durchbrechung 44 ist hohlzylindrisch oder im Wesentlichen hohlzylindrisch ausgebildet.

Bei anderen Ausführungsbeispielen weist die Durchbrechung 44 einen ovalen oder rechteckigen, insbesondere quadratischen, Querschnitt auf.

Die Durchbrechung 44 definiert eine Durchbrechungslängsachse 46, welche quer zur ersten Wirbelkörperanlagefläche 24 und quer zur zweiten Wirbelkörperanlagefläche 26 verläuft. Bei dem in den Figuren 1 bis 4 dargestellten Ausführungsbeispiel des Zwischenwirbelimplantats 10 verläuft die Durchbrechungslängsachse 46 senkrecht zur zweiten Wirbelkörperanlagefläche 26 und senkrecht zur Tangentialebene 34 an die vom Zwischenwirbelimplantat 10 weg weisend konvex gekrümmte erste Wirbelkörperanlagefläche 24.

Die beiden inneren Rahmenteile 36 und 38 begrenzen die Durchbrechung 44 abschnittsweise.

Die Stützelemente 30 halten die Rahmenteile 36 und 38 in einem definierten Abstand 48 voneinander.

Das Zwischenwirbelimplantat 10 ist insgesamt spiegelsymmetrisch bezogen auf eine Spiegelebene 50 ausgebildet, welche sich senkrecht zur ersten Wirbelkörperanlagefläche 24 erstreckt.

Die Rahmenstruktur 28 umfasst ferner einen in sich geschlossenen ersten äußeren Rahmenteil 52 und einen zweiten in sich geschlossenen äußeren Rahmenteil 54. Die äußeren Rahmenteile 52 und 54 sind mit den inneren Rahmenteilen 36 und 38 verbunden. Die Rahmenstruktur 28 ist insgesamt verwindungssteif ausgebildet.

Der erste äußere Rahmenteil 52 begrenzt die Implantatoberseite 20 abschnittsweise beziehungsweise bildet einen Teil der Implantatoberseite 20. In analoger Weise begrenzt der zweite äußere Rahmenteil 54 die Implantatunterseite 22 abschnittsweise beziehungsweise bildet einen Teil derselben.

Die Rahmenteile 52 und 54 sind in Form jeweils eines Rings 56 beziehungsweise 58 ausgebildet. Die Ringe 56 und 58 sind zwar in sich geschlossen, jedoch nicht kreisförmig ausgebildet. Sie sind aus mehreren Abschnitten mit unterschiedlichen Krümmungsradien zusammengesetzt ausgebildet, wobei an eine Rückseite 60 des Zwischenwirbelimplantats 10 angrenzende Abschnitte der Ringe 56 und 58 geradlinig verlaufen, wobei diese Abschnitte mit von ihnen definierten Längsachsen senkrecht zu den Stützelementlängsachsen 32 und parallel zur zweiten Wirbelkörperanlagefläche 26 verlaufen.

Die Rahmenstruktur 28 umfasst ferner einen Rahmengrundkörper 62, welcher massiv ausgebildet ist und die äußeren Rahmenteile 52 und 54 mit den inneren Rahmenteilen 36, 38 verbindet.

Der Rahmengrundkörper 62 ist im Wesentlichen quaderförmig ausgebildet und erstreckt sich von einer plattenförmigen Vorderseite 64 bis zur Durchbrechung 44, und zwar von der Implantatoberseite 20 bis zur Implantatunterseite 22.

Das Zwischenwirbelimplantat 10 umfasst ferner eine Instrumentenaufnahme 66 zum kraft- und/oder formschlüssigen in Eingriff Bringen mit einem in den Figuren nicht dargestellten Einsetzinstrument.

Die Instrumentenaufnahme 66 ist am Rahmengrundkörper 62 angeordnet beziehungsweise ausgebildet und definiert eine Instrumentenaufnahmelängsachse 68, die quer, bei dem in den Figuren 1 bis 4 dargestellten Ausführungsbeispiel des Zwischenwirbelimplantats 10 senkrecht, zur Durchbrechungslängsachse 46 verläuft.

Die Instrumentenaufnahme 66 ist in Form einer Bohrung 70 ausgebildet. Bei einem weiteren Ausführungsbeispiel ist die Instrumentenaufnahme durch eine Hülse ausgebildet. Ferner ist die Instrumentenaufnahme 66 mit einem Innengewinde 72 versehen.

Die Vorderseite 64 des Zwischenwirbelimplantats 10 erstreckt sich quer zur ersten Wirbelkörperanlagefläche 24, insbesondere senkrecht zur Tangentialebene 34, und quer, nämlich senkrecht, zur zweiten Wirbelkörperanlagefläche 26.

Die Instrumentenaufnahme 66 erstreckt sich von der Vorderseite 64 bis zur Durchbrechung 44, und zwar parallel zur Implantatunterseite 22.

Die erste Wirbelkörperanlagefläche 24 ist abschnittsweise eben ausgebildet. Die zweite Wirbelkörperanlagefläche 26 ist vollständig eben ausgebildet.

Die erste Wirbelkörperanlagefläche 24 definiert eine erste Anlageebene 74, die sich ausgehend von der Rückseite 60 auf einem kurzen Abschnitt der Implantatoberseite 20 in Richtung zur Vorderseite 64 hin erstreckt.

Die zweite Wirbelkörperanlagefläche 26 definiert eine zweite Anlageebene 76. Bei dem dargestellten Ausführungsbeispiel des Zwischenwirbelimplantats 10 sind die Anlageebenen 74 und 76 um einen Neigungswinkel 78 gegeneinander geneigt. Der Neigungswinkel 78 weist einen Wert in einem Bereich zwischen 0° und etwa 20° auf.

Die erste Wirbelkörperanlagefläche 24 ist abschnittsweise vom Zwischenwirbelimplantat 10 weg weisend konvex gekrümmt, und zwar zwei- beziehungsweise dreidimensional gekrümmt.

Die Stützelemente 30 weisen vom Zwischenwirbelimplantat 10 weg weisende Endflächen 80 und 82 auf. Die Endflächen 80 und 82 bilden einen Teil der ersten Wirbelkörperanlagefläche 24 beziehungsweise der zweiten Wirbelkörperanlagefläche 26.

Auf den Endflächen 80 und 82 ist jeweils ein Verankerungsvorsprung 84 beziehungsweise 86 angeordnet. Die Verankerungsvorsprünge 84 und 86 sind spitz ausgebildet und definieren eine Spitze 88 beziehungsweis 90. Insgesamt sind die Verankerungsvorsprünge 84 und 86 kegelförmig oder im Wesentlichen kegelförmig ausgebildet.

Die Verankerungsvorsprünge 84 und 86 definieren Vorsprunglängsachsen 92, die parallel zu den Stützelementlängsachsen 32 der zugeordneten Stützelemente 30 verlaufen.

Ein Querschnitt der Stützelemente 30 bezogen auf die jeweilige Stützelementlängsachse 32 ist durch eine Kombination aus einem Rechteck und einem Halbkreis gebildet. Der Halbkreis ist von der Durchbrechung 44 weg weisend in Richtung auf die äußeren Rahmenteile 52 und 54 gerichtet. Die Verankerungsvorsprünge 84 und 86 sind mit ihrer Grundfläche auf den Endflächen 80 und 82 so angeordnet beziehungsweise ausgerichtet, dass der Halbkreis die Hälfte der jeweiligen Grundfläche überdeckt.

Die Stützelemente 30 sind insgesamt jeweils massiv ausgebildet. Sie weisen keine Hohlräume auf.

Das Zwischenwirbelimplantat 10 sowie die Rahmenstruktur 28 sind durch ein additives Herstellungsverfahren ausgebildet. Ist das Zwischenwirbelimplantat 10 aus einem metallischen Werkstoff ausgebildet, ist es additiv beispielsweise durch selektives Lasersintern oder Elektronenstrahlschmelzen ausgebildet. Ein aus einem Kunststoff ausgebildetes Zwischenwirbelimplantat 10 ist beispielsweise durch 3D-Druck ausgebildet.

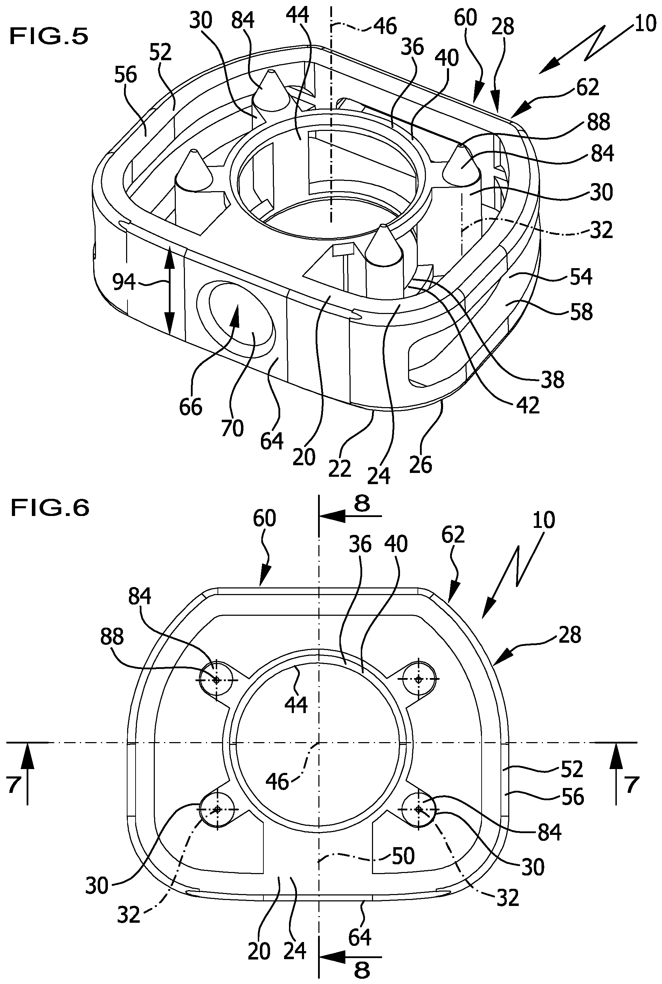

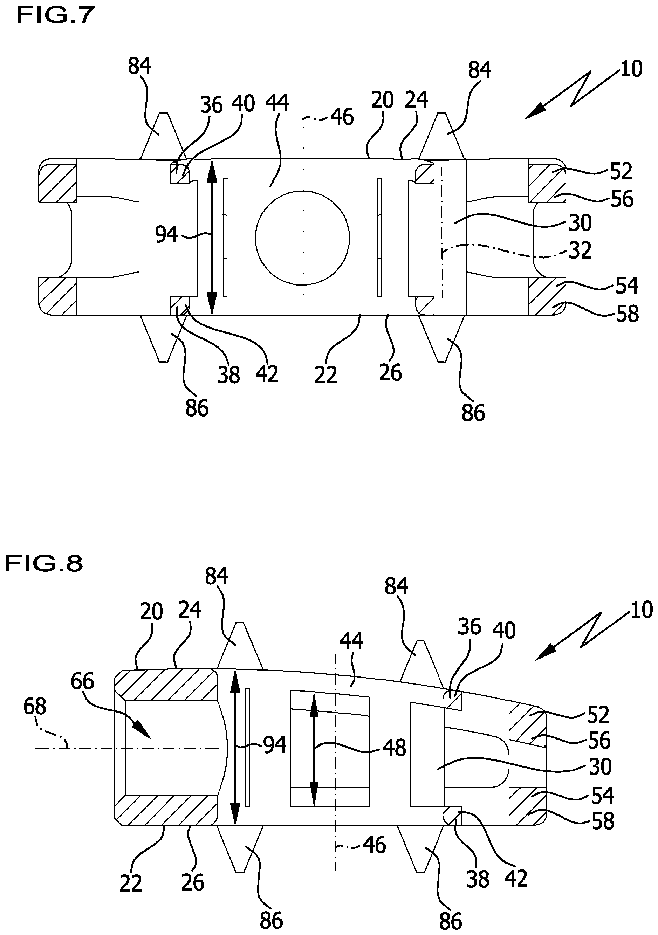

In den Figuren 5 bis 8 ist ein weiteres Ausführungsbeispiel eines ebenfalls mit dem Bezugszeichen 10 bezeichneten Zwischenwirbelimplantats schematisch dargestellt. Es stimmt in seinem Aufbau grundsätzlich mit dem in den Figuren 1 bis 4 dargestellten Zwischenwirbelimplantat überein, sodass hier zur Bezeichnung einzelner Elemente dieselben Bezugszeichen wie bei dem in den Figuren 1 bis 4 dargestellten Ausführungsbeispiel verwendet wurden.

Das in den Figuren 5 bis 8 dargestellte Ausführungsbeispiel unterscheidet sich lediglich aufgrund seiner Abmessungen von dem in den Figuren 1 bis 4 dargestellten Ausführungsbeispiel. Deutlich wird dies insbesondere in der unterschiedlichen Höhe 94, die einen Abstand zwischen der ersten Wirbelkörperanlagefläche 24 und der zweiten Wirbelkörperanlagefläche 26 im Bereich der Vorderseite 64 definiert.

Besonders deutlich wird der Größenunterschied der beiden in den Figuren 1 bis 8 dargestellten Ausführungsbeispiele der Zwischenimplantate 10, wenn berücksichtigt wird, dass die Instrumentenaufnahme 66 bei beiden Ausführungsbeispielen identisch dimensioniert ist. So ergibt sich insbesondere direkt, dass die Höhe 94 beim Ausführungsbeispiel der Figuren 5 bis 8 nur etwa knapp 40% der Höhe 94 beim Ausführungsbeispiel der Figuren 1 bis 4 beträgt.

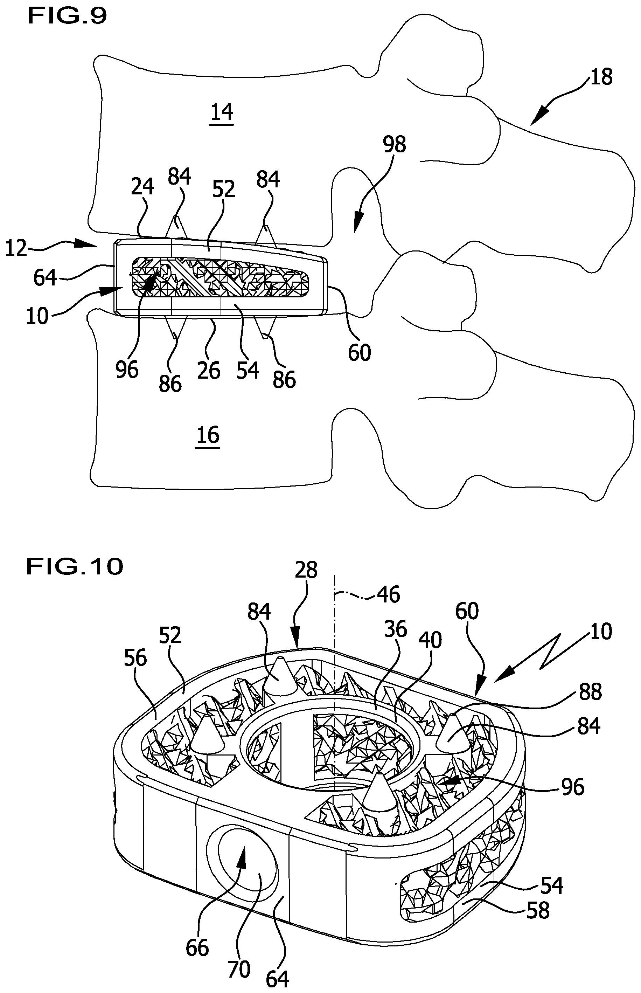

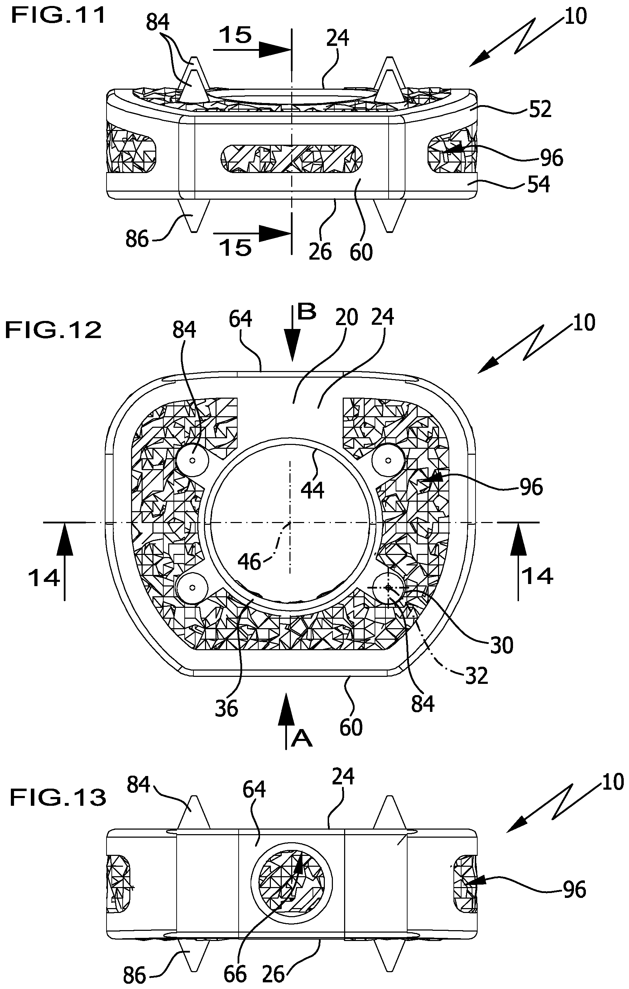

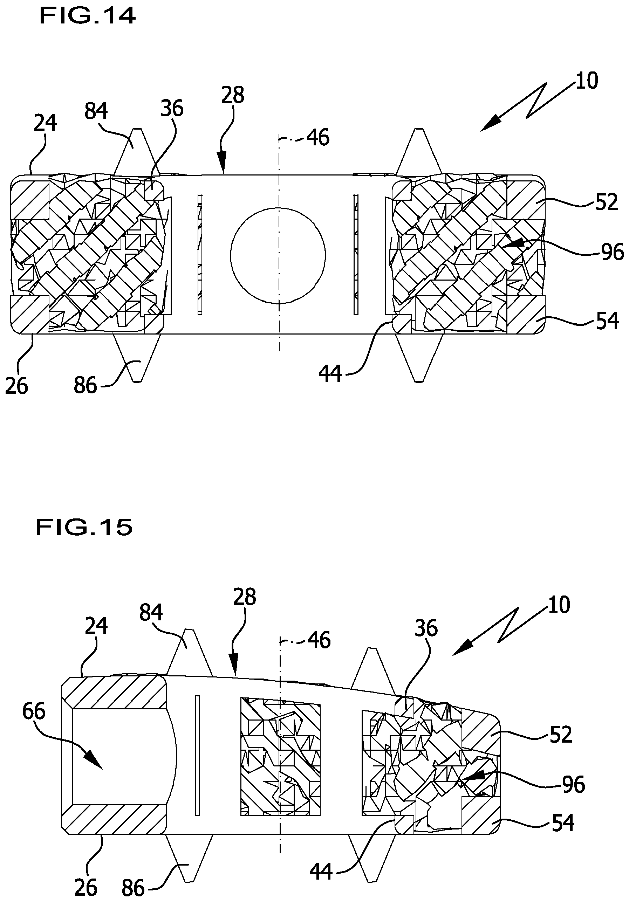

In den Figuren 9 bis 15 ist ein weiteres Ausführungsbeispiel eines insgesamt mit dem Bezugszeichen 10 bezeichneten Zwischenwirbelimplantats schematisch dargestellt. Es stimmt in seinem Aufbau vollständig mit dem Ausführungsbeispiel der Figuren 5 bis 8 überein. Zusätzlich ist das vom Zwischenwirbelimplantat 10 definierte Implantatvolumen bis auf die Durchbrechung 44 und die Instrumentenaufnahme 66 mit einer offenporigen Gitterstruktur 96 ausgefüllt.

Die Gitterstruktur 96 weist eine Vielzahl von miteinander in Fluidverbindung stehender Hohlräume auf, in die Knochengewebe des implantierten Zwischenwirbelimplantats einwachsen kann.

Figur 9 zeigt schematisch das Zwischenwirbelimplantat 10, das in den Bandscheibenraum 12 zwischen die Wirbelkörper 14 und 16 eingesetzt ist. Die Verankerungsvorsprünge 84 und 86 dringen in die Wirbelkörper 14 und 16 ein und verhindern eine Bewegung des Zwischenwirbelimplantats 10 insbesondere in einer Richtung parallel zu den Wirbelkörperanlageflächen 24 beziehungsweise 26 und damit insbesondere auch in Richtung auf einen Spinalkanal 98 der Wirbelsäule 18 hin.

Das Zwischenwirbelimplantat 10 wird in das Bandscheibenfach 12, also den Zwischenwirbelraum zwischen den Wirbelkörpern 14 und 16 derart eingesetzt, dass die Vorderseite 64 in distaler beziehungsweise anteriorer Richtung weist, also in Richtung auf beispielsweise einen Bauchraum eines Patienten hin, die Rückseite 60 in dorsaler Richtung, also in Richtung auf den Spinalkanal 98 hin, wenn es sich bei den Wirbelkörpern 14 und 16 um Wirbelkörper von Lendenwirbeln handelt.

Das Zwischenwirbelimplantat 10 gemäß dem Ausführungsbeispiel, das in den Figuren 1 bis 4 dargestellt ist, kann ebenfalls mit einer in Verbindung mit dem Ausführungsbeispiel der Figuren 9 bis 15 beschriebenen Gitterstruktur 96 ausgefüllt sein, und zwar insbesondere vollständig oder lediglich im Bereich des vom Zwischenwirbelimplantat 10 definierten Implantatvolumens mit Ausnahme der Durchbrechung 44 und der Instrumentenaufnahme 66.

Alle beschriebenen Ausführungsbeispiele von Zwischenwirbelimplantaten 10 haben insbesondere gemeinsam, dass die Verankerungsvorsprünge 84 und 86 solide an die Rahmenstruktur 28 angebunden sind. Sie können also nicht in das vom jeweiligen Zwischenwirbelimplantat 10 definierte Volumen hineingedrückt werden, wie dies der Fall wäre, wenn die Verankerungsvorsprünge im Bereich, also direkt an der Gitterstruktur 96 angeordnet würden. Ein Abscheren oder ein sogenanntes Versenken der Verankerungsvorsprünge im Implantatvolumen kann so vermieden werden. Insgesamt kann eine deutlich verbesserte Stabilität des Zwischenwirbelimplantats mit hervorragenden Eigenschaften zum Einwachsen von Knochengewebe in das Zwischenwirbelimplantat erreicht werden.

Die massive Ausgestaltung der Stützelemente, die Sockel für die Verankerungsvorsprünge 84 und 86 bilden, erleichtert die Reinigung der Gitterstruktur 96. Insbesondere das Auswaschen der Zwischenwirbelimplantate 10 nach der Fertigung, insbesondere zur Entfernung von Hilfsmittelrückständen, wird verbessert, da Spülschatten durch die Verankerungsvorsprünge 84 und 86 weitestgehend reduziert wurden. Spülschatten können insbesondere dann entstehen, wenn die Verankerungsvorsprünge direkt auf die Gitterstruktur 96 aufgesetzt würden, sodass hinter den Verankerungsvorsprüngen 84 und 86 Hohlräume verbleiben würden, die nur schwer reinigbar sind.

### Bezugszeichenliste

- 10: Zwischenwirbelimplantat
- 12: Bandscheibenraum
- 14: Wirbelkörper
- 16: Wirbelkörper
- 18: Wirbelsäule
- 20: Implantatoberseite
- 22: Implantatunterseite
- 24: erste Wirbelkörperanlagefläche
- 26: zweite Wirbelkörperanlagefläche
- 28: Rahmenstruktur
- 30: Stützelement
- 32: Stützelementlängsachse
- 34: Tangentialebene
- 36: erster innerer Rahmenteil
- 38: zweiter innerer Rahmenteil
- 40: Ring
- 42: Ring
- 44: Durchbrechung
- 46: Durchbrechungslängsachse
- 48: Abstand
- 50: Spiegelebene
- 52: erster äußerer Rahmenteil
- 54: zweiter äußerer Rahmenteil
- 56: Ring
- 58: Ring
- 60: Rückseite
- 62: Rahmengrundkörper
- 64: Vorderseite
- 66: Instrumentenaufnahme
- 68: Instrumentenaufnahmelängsachse
- 70: Bohrung
- 72: Innengewinde
- 74: Anlageebene
- 76: Anlageebene
- 78: Neigungswinkel
- 80: Endfläche
- 82: Endfläche
- 84: Verankerungsvorsprung
- 86: Verankerungsvorsprung
- 88: Spitze
- 90: Spitze
- 92: Vorsprunglängsachse
- 94: Höhe
- 96: Gitterstruktur
- 98: Spinalkanal

## Patentansprüche

1. Zwischenwirbelimplantat (10) zum Einsetzen in einen Bandscheibenraum (12) zwischen zwei benachbarte Wirbelkörper (14, 16) einer menschlichen oder tierischen Wirbelsäule (18), wobei das Zwischenwirbelimplantat eine Implantatoberseite (20), welche eine erste Wirbelkörperanlagefläche (24) zum Anlegen an einen ersten Wirbelkörper (14) definiert, und eine Implantatunterseite (22), welche eine zweite Wirbelkörperanlagefläche (26) zum Anlegen an einen zweiten Wirbelkörper (16) definiert, aufweist, wobei das Zwischenwirbelimplantat (10) eine Rahmenstruktur (28) mit mindestens zwei Stützelementen (30) umfasst, wobei sich die mindestens zwei Stützelemente (30) von der Implantatoberseite (20) bis zur Implantatunterseite (22) erstrecken und wobei die mindestens zwei Stützelemente (30) Stützelementlängsachsen (32) definieren, die quer, insbesondere senkrecht, zur ersten und/oder zweiten Wirbelkörperanlagenfläche (24, 26) verlaufen, **dadurch gekennzeichnet, dass** die Rahmenstruktur (28) einen ersten in sich geschlossenen inneren Rahmenteil (36) und einen zweiten in sich geschlossenen inneren Rahmenteil (38) umfasst und dass die mindestens zwei Stützelemente (30) die beiden inneren Rahmenteile (36, 38) miteinander verbinden durch eine direkte Anbindung der inneren Rahmenteile (36, 38) an die mindestens zwei Stützelemente (30).

2. Zwischenwirbelimplantat nach Anspruch 1, **dadurch gekennzeichnet, dass** die mindestens zwei Stützelemente (30) vom Zwischenwirbelimplantat (10) weg weisende Endflächen (80, 82) aufweisen und dass mindestens eine dieser Endflächen (80, 82) mindestens einen vom Zwischenwirbelimplantat (10) weg weisenden Verankerungsvorsprung (84, 86) trägt,
wobei insbesondere der mindestens eine Verankerungsvorsprung (84, 86)
a) spitz, insbesondere dornartig,
und/oder
b) kegelförmig oder im Wesentlichen kegelförmig ausgebildet ist.

3. Zwischenwirbelimplantat nach Anspruch 2, **dadurch gekennzeichnet, dass** der mindestens eine Verankerungsvorsprung (84, 86) eine Vorsprunglängsachse (92) definiert und dass die Vorsprunglängsachse (92) parallel oder im Wesentlichen parallel zur Stützelementlängsachse (32) des zugeordneten Stützelements (30) verläuft.

4. Zwischenwirbelimplantat nach einem der voranstehenden Ansprüche, **dadurch gekennzeichnet, dass** die mindestens zwei Stützelemente (30) einen Querschnitt bezogen auf die jeweilige Stützelementlängsachse (32) definieren, welcher rechteckig, dreieckig oder kreisförmig oder durch eine Kombination aus einem Rechteck und einem Halbkreis gebildet ist.

5. Zwischenwirbelimplantat nach einem der voranstehenden Ansprüche, **dadurch gekennzeichnet, dass**
a) die mindestens zwei Stützelemente (30) massiv ausgebildet sind
und/oder
b) das Zwischenwirbelimplantat (10) eine zentrale Durchbrechung (44) aufweist und dass sich die Durchbrechung (44) von der Implantatoberseite (20) bis zur Implantatunterseite (22) durch das Zwischenwirbelimplantat (10) hindurch erstreckt,
wobei insbesondere die Durchbrechung (44)
hohlzylindrisch oder im Wesentlichen hohlzylindrisch ausgebildet ist oder einen ovalen oder rechteckigen, insbesondere quadratischen, Querschnitt aufweist
und/oder
eine Durchbrechungslängsachse (46) definiert und dass die Durchbrechungslängsachse (46) quer, insbesondere senkrecht, zur ersten Wirbelkörperanlagefläche (24) und/oder quer, insbesondere senkrecht, zur zweiten Wirbelkörperanlagefläche (26) verläuft.

6. Zwischenwirbelimplantat nach einem der voranstehenden Ansprüche, **dadurch gekennzeichnet, dass** das Zwischenwirbelimplantat (10) eine zentrale Durchbrechung (44) aufweist, dass sich die Durchbrechung (44) von der Implantatoberseite (20) bis zur Implantatunterseite (22) durch das Zwischenwirbelimplantat (10) hindurch erstreckt und dass die beiden inneren Rahmenteile (36, 38) die Durchbrechung (44) mindestens abschnittsweise begrenzen.

7. Zwischenwirbelimplantat nach einem der voranstehenden Ansprüche, **dadurch gekennzeichnet, dass** der erste innere Rahmenteil (36) in Form eines Rings (40) ausgebildet ist und/oder dass der zweite innere Rahmenteil (38) in Form eines Rings (42) ausgebildet ist.

8. Zwischenwirbelimplantat nach einem der voranstehenden Ansprüche, **dadurch gekennzeichnet, dass** der erste innere Rahmenteil (36) die Implantatoberseite (20) mindestens abschnittsweise begrenzt und/oder dass der zweite innere Rahmenteil (38) die Implantatunterseite (22) mindestens abschnittsweise begrenzt.

9. Zwischenwirbelimplantat nach einem der voranstehenden Ansprüche, **dadurch gekennzeichnet, dass** die Rahmenstruktur (28) einen ersten in sich geschlossenen äußeren Rahmenteil (52) und einen zweiten in sich geschlossenen äußeren Rahmenteil (54) umfasst und dass die beiden äußeren Rahmenteile (52, 54) mit den beiden inneren Rahmenteilen (36, 38), insbesondere verwindungssteif, verbunden sind.

10. Zwischenwirbelimplantat nach Anspruch 9, **dadurch gekennzeichnet, dass** der erste äußere Rahmenteil (52) die Implantatoberseite (20) mindestens abschnittsweise begrenzt und/oder dass der zweite äußere Rahmenteil (54) die Implantatunterseite (22) mindestens abschnittsweise begrenzt.

11. Zwischenwirbelimplantat nach Anspruch 9 oder 10, **dadurch gekennzeichnet, dass** der erste äußere Rahmenteil (52) in Form eines Rings (56) ausgebildet ist und/oder dass der zweite äußere Rahmenteil (54) in Form eines Rings (58) ausgebildet ist.

12. Zwischenwirbelimplantat nach einem der Ansprüche 9 bis 11, **dadurch gekennzeichnet, dass** das Zwischenwirbelimplantat (10) einen Rahmengrundkörper (62) umfasst und dass der Rahmengrundkörper (62) die äußeren Rahmenteile (52, 54) mit den inneren Rahmenteilen (36, 38) verbindet,
wobei insbesondere der Rahmengrundkörper (62)
a) massiv
und/oder
b) quaderförmig oder im Wesentlichen quaderförmig ausgebildet ist.

13. Zwischenwirbelimplantat nach einem der voranstehenden Ansprüche, **dadurch gekennzeichnet, dass** das Zwischenwirbelimplantat (10) eine Instrumentenaufnahme (66) zum kraft- und/oder formschlüssigen in Eingriff Bringen mit einem Einsetzinstrument umfasst,
wobei insbesondere die Instrumentenaufnahme (66) am Rahmengrundkörper (62) angeordnet oder ausgebildet ist.

14. Zwischenwirbelimplantat nach einem der voranstehenden Ansprüche, **dadurch gekennzeichnet, dass** ein vom Zwischenwirbelimplantat (10) definiertes Implantatvolumen mindestens teilweise durch eine offenporige Gitterstruktur (96) ausgefüllt ist, insbesondere vollständig,
wobei insbesondere die Gitterstruktur (96) durch ein generatives Herstellungsverfahren ausgebildet ist.

15. Zwischenwirbelimplantat nach einem der voranstehenden Ansprüche, **dadurch gekennzeichnet, dass** die mindestens zwei Stützelemente (30) eine Querschnittsfläche definieren, die längs ihrer Streckung konstant oder im Wesentlichen konstant ist.

## Claims

1. Intervertebral implant (10) for insertion into an intervertebral disc space (12) between two adjacent vertebral bodies (14, 16) of a human or animal spine (18), wherein the intervertebral implant has an implant top side (20), which defines a first vertebral body abutment face (24) for abutting against a first vertebral body (14), and an implant bottom side (22), which defines a second vertebral body abutment face (26) for abutting against a second vertebral body (16), wherein the intervertebral implant (10) comprises a frame structure (28) with at least two support elements (30), wherein the at least two support elements (30) extend from the implant top side (20) to the implant bottom side (22), and wherein the at least two support elements (30) define support element longitudinal axes (32), which run transversely, in particular perpendicularly, to the first and/or second vertebral body abutment face (24, 26), **characterized in that** the frame structure (28) comprises a first self-enclosed inner frame part (36) and a second self-enclosed inner frame part (38), and **in that** the at least two support elements (30) connect the two inner frame parts (36, 38) to one another by a direct connection of the inner frame parts (36, 38) to the at least two support elements (30).

2. Intervertebral implant in accordance with Claim 1, **characterized in that** the at least two support elements (30) have end faces (80, 82) that face away from the intervertebral implant (10) and **in that** at least one of these end faces (80, 82) bears at least one anchoring projection (84, 86) pointing away from the intervertebral implant (10),
wherein, in particular, the at least one anchoring projection (84, 86)
a) is of pointed, in particular thorn-like, configuration
and/or
b) is of conical or substantially conical configuration.

3. Intervertebral implant in accordance with Claim 2, **characterized in that** the at least one anchoring projection (84, 86) defines a projection longitudinal axis (92) and **in that** the projection longitudinal axis (92) runs in parallel or substantially in parallel to the support element longitudinal axis (32) of the associated support element (30).

4. Intervertebral implant in accordance with any one of the preceding Claims, **characterized in that** the at least two support elements (30) define a cross section in relation to the respective support element longitudinal axis (32), which is rectangular, triangular, or circular, or is formed by a combination of a rectangle and a semicircle.

5. Intervertebral implant in accordance with any one of the preceding Claims, **characterized in that**
a) the at least two support elements (30) are of solid configuration,
and/or
b) the intervertebral implant (10) has a central perforation (44) and **in that** the perforation (44) extends from the implant top side (20) to the implant bottom side (22) through the intervertebral implant (10),
wherein, in particular, the perforation (44)
is of hollow-cylindrical or substantially hollow-cylindrical configuration or has an oval or rectangular, in particular square, cross section,
and/or
defines a perforation longitudinal axis (46) and **in that** the perforation longitudinal axis (46) runs transversely, in particular perpendicularly, to the first vertebral body abutment face (24) and/or transversely, in particular perpendicularly, to the second vertebral body abutment face (26).

6. Intervertebral implant in accordance with any one of the preceding Claims, **characterized in that** the intervertebral implant (10) has a central perforation (44), **in that** the perforation (44) extends from the implant top side (20) to the implant bottom side (22) through the intervertebral implant (10), and **in that** the two inner frame parts (36, 38) delimit the perforation (44) at least in sections.

7. Intervertebral implant in accordance with any one of the preceding Claims, **characterized in that** the first inner frame part (36) is configured in the form of a ring (40) and/or **in that** the second inner frame part (38) is configured in the form of a ring (42).

8. Intervertebral implant in accordance with any one of the preceding Claims, **characterized in that** the first inner frame part (36) delimits the implant top side (20) at least in sections and/or **in that** the second inner frame part (38) delimits the implant bottom side (22) at least in sections.

9. Intervertebral implant in accordance with any one of the preceding Claims, **characterized in that** the frame structure (28) comprises a first self-enclosed outer frame part (52) and a second self-enclosed outer frame part (54), and **in that** the two outer frame parts (52, 54) are connected, in particular in a torsionally resistant manner, to the two inner frame parts (36, 38).

10. Intervertebral implant in accordance with Claim 9, **characterized in that** the first outer frame part (52) delimits the implant top side (20) at least in sections and/or **in that** the second outer frame part (54) delimits the implant bottom side (22) at least in sections.

11. Intervertebral implant in accordance with Claim 9 or 10, **characterized in that** the first outer frame part (52) is configured in the form of a ring (56) and/or **in that** the second outer frame part (54) is configured in the form of a ring (58).

12. Intervertebral implant in accordance with any one of Claims 9 to 11, **characterized in that** the intervertebral implant (10) comprises a frame base body (62) and **in that** the frame base body (62) connects the outer frame parts (52, 54) to the inner frame parts (36, 38),
wherein, in particular, the frame base body (62)
a) is of solid configuration
and/or
b) is of cuboidal or substantially cuboidal configuration.

13. Intervertebral implant in accordance with any one of the preceding Claims, **characterized in that** the intervertebral implant (10) comprises an instrument receptacle (66) for being brought into force-locking and/or positive-locking engagement with an insertion instrument,
wherein, in particular, the instrument receptacle (66) is arranged or formed on the frame base body (62).

14. Intervertebral implant in accordance with any one of the preceding Claims, **characterized in that** an implant volume defined by the intervertebral implant (10) is at least partially, in particular completely, filled by an open-pored lattice structure (96),
wherein, in particular, the lattice structure (96) is produced by a generative production method.

15. Intervertebral implant in accordance with any one of the preceding Claims, **characterized in that** the at least two support elements (30) define a cross sectional area that is constant or substantially constant along its extent.

## Revendications

1. Implant intervertébral (10) destiné à être inséré dans un espace discal (12) entre deux corps vertébraux (14, 16) adjacents d'une colonne vertébrale (18) humaine ou animale, dans lequel l'implant intervertébral présente une face supérieure d'implant (20), qui définit une première surface d'appui de corps vertébral (24) pour l'appui contre un premier corps vertébral (14) et une face inférieure d'implant (22), qui définit une deuxième surface d'appui de corps vertébral (26) pour l'appui contre un deuxième corps vertébral (16), dans lequel l'implant intervertébral (10) comprend une structure de cadre (28) avec au moins deux éléments de soutien (30), dans lequel les au moins deux éléments de soutien (30) s'étendent de la face supérieure d'implant (20) à la face inférieure d'implant (22) et dans lequel les au moins deux éléments de soutien (30) définissent des axes longitudinaux d'éléments de soutien (32), qui s'étendent transversalement, plus particulièrement perpendiculairement, par rapport à la première et/ou à la deuxième surface d'appui de corps vertébral (24, 26), **caractérisé en ce que** la structure de cadre (28) comprend une première partie de cadre interne (36) fermée sur elle-même et une deuxième partie de cadre interne (38) fermée sur elle-même et **en ce que** les au moins deux éléments de soutien (30) relient entre elles les deux parties de cadre internes (36, 38) à l'aide d'une liaison directe des parties de cadre internes (36, 38) aux au moins deux éléments de soutien (30).

2. Implant intervertébral selon la revendication 1, **caractérisé en ce que** les au moins deux éléments de soutien (30) présentent des surfaces d'extrémité (80, 82) opposées à l'implant intervertébral (10) et **en ce qu'**au moins une de ces surfaces d'extrémité (80, 82) supporte au moins une saillie d'ancrage (84, 86) opposée à l'implant intervertébral (10), dans lequel, plus particulièrement, l'au moins une saillie d'ancrage (84, 86) est réalisée
a) avec une forme pointue, plus particulièrement sous la forme d'une broche,
et/ou
b) avec une forme conique ou globalement conique.

3. Implant intervertébral selon la revendication 2, **caractérisé en ce que** l'au moins une saillie d'ancrage (84, 86) définit un axe longitudinal de saillie (92) et **en ce que** l'axe longitudinal de saillie (92) s'étend parallèlement ou globalement parallèlement par rapport à l'axe longitudinal d'élément de soutien (32) de l'élément de soutien (30) correspondant.

4. Implant intervertébral selon l'une des revendications précédentes, **caractérisé en ce que** les au moins deux éléments de soutien (30) définissent une section transversale par rapport à l'axe longitudinal d'élément de soutien (32), qui présente une forme rectangulaire, triangulaire ou circulaire ou est constituée d'une combinaison d'un rectangle et d'un demi-cercle.

5. Implant intervertébral selon l'une des revendications précédentes, **caractérisé en ce que**
a) les au moins deux éléments de soutien (30) sont réalisés de manière massive
et/ou
b) l'implant intervertébral (10) présente un perçage central (44) et **en ce que** le perçage (44) s'étend de la face supérieure de l'implant (20) jusqu'à la face inférieure de l'implant (22) à travers l'implant intervertébral (10),
dans lequel, plus particulièrement, le perçage (44),
présente une forme cylindrique creuse ou globalement cylindrique creuse ou présente une section transversale ovale ou rectangulaire, plus particulièrement carrée,
et/ou
définit un axe longitudinal de perçage (46) et **en ce que** l'axe longitudinal de perçage (46) s'étend transversalement, plus particulièrement perpendiculairement, par rapport à la première surface d'appui de corps vertébral (24) et/ou transversalement, plus particulièrement perpendiculairement, par rapport à la deuxième surface d'appui du corps vertébral (26).

6. Implant intervertébral selon l'une des revendications précédentes, **caractérisé en ce que** l'implant intervertébral (10) présente un perçage central (44), **en ce que** le perçage central (44) s'étend de la face supérieure d'implant (20) à la face inférieure d'implant (22), à travers l'implant intervertébral (10) et **en ce que** les deux parties de cadre internes (36, 38) délimitent le perçage (44) au moins à certains endroits.

7. Implant intervertébral selon l'une des revendications précédentes, **caractérisé en ce que** la première partie de cadre interne (36) présente la forme d'un anneau (40) et/ou **en ce que** la deuxième partie de cadre interne (38) présente la forme d'un anneau (42).

8. Implant intervertébral selon l'une des revendications précédentes, **caractérisé en ce que** la première partie de cadre interne (36) délimite la face supérieure d'implant (20) au moins à certains endroits et/ou **en ce que** la deuxième partie de cadre interne (38) délimite la face inférieure d'implant (22) au moins à certains endroits.

9. Implant intervertébral selon l'une des revendications précédentes, **caractérisé en ce que** la structure de cadre (28) comprend une première partie de cadre externe (52) fermée sur elle-même et une deuxième partie de cadre externe (54) fermée sur elle-même et **en ce que** les deux parties de cadre externes (52, 54) sont reliées avec les deux parties de cadre internes (36, 38), plus particulièrement de manière résistante à la torsion.

10. Implant intervertébral selon la revendication 9, **caractérisé en ce que** la première partie de cadre externe (52) délimite la face supérieure d'implant (20) au moins à certains endroits et/ou **en ce que** la deuxième partie de cadre externe (54) délimite la face inférieure d'implant (22) au moins à certains endroits.

11. Implant intervertébral selon la revendication 9 ou 10, **caractérisé en ce que** la première partie de cadre externe (52) présente la forme d'un anneau (56) et/ou **en ce que** la deuxième partie de cadre externe (54) présente la forme d'un anneau (58).

12. Implant intervertébral selon l'une des revendications 9 à 11, **caractérisé en ce que** l'implant intervertébral (10) comprend un corps de base de cadre (62) et **en ce que** le corps de base de cadre (62) relie les parties de cadre externes (52, 54) avec les parties de cadre internes (36, 38), dans lequel, plus particulièrement, le corps de base de cadre (62) est réalisé
a) de manière massive
et/ou
b) sous une forme parallélépipédique ou globalement parallélépipédique.

13. Implant intervertébral selon l'une des revendications précédentes, **caractérisé en ce que** l'implant intervertébral (10) comprend un logement d'instrument (66) pour l'emboîtement par force et/ou par complémentarité de forme avec un instrument d'insertion,
dans lequel, plus particulièrement, le logement d'instrument (66) est disposé ou réalisé sur le corps de base de cadre (62).

14. Implant intervertébral selon l'une des revendications précédentes, **caractérisé en ce qu'**un volume d'implant défini par l'implant intervertébral (10) est rempli au moins partiellement, plus particulièrement de manière complète, par une structure de grille à pores ouverts (96),
dans lequel, plus particulièrement la structure de grille (96) est réalisé à l'aide d'un procédé de fabrication génératif.

15. Implant intervertébral selon l'une des revendications précédentes, **caractérisé en ce que** les au moins deux éléments de soutien (30) définissent une surface de section transversale qui est constante ou globalement constante le long de son extension.
